# EUROPEAN PATENT SPECIFICATION

(11) **EP 4 561 455 B1**
(45) Date of publication and mention of the grant of the patent: **22.04.2026**
(21) Application number: 23751276.9
(22) Date of filing: 27.07.2023
(51) Int. Cl.: A61B 17/04, A61B 17/00, A61B 17/06

(54) **TISSUE CLOSURE DEVICE**
GEWEBEVERSCHLUSSVORRICHTUNG
DISPOSITIF DE FERMETURE DE TISSU

(30) Priority: 28.07.2022 CN 202210900379
(43) Date of publication of application: 04.06.2025
(73) Proprietor: Cilag GmbH International, 6300 Zug (CH)
(72) Inventor: YANG, Mengli, Shanghai (CN); DING, Sheng, Shanghai (CN); LIU, Qinglong, Shanghai (CN); ZHANG, Jie, Shanghai (CN)
(74) Representative: van Wanrooij, Eva
(86) International application number: PCT/EP2023/070812
(87) International publication number: WO 2024/023201

(56) References cited:
- EP-A1- 3 473 189
- CN-A- 111 374 722
- US-A1- 2002 005 204

## Description

### TECHNICAL FIELD

The present invention relates to the field of surgical instruments, and in particular to a tissue closure device.

### BACKGROUND OF THE INVENTION

For the existing tissue closure devices in surgical instruments, generally, the main body of the closure device is inserted into the human body or animal body tissue first, and then a suture is carried by an access needle into the tissue. After entering the tissue, the suture is fixed on a distal structure of the tissue closure device, and suturing is then completed. Because the access needle is operated outside the tissue closure device, the access needle is likely to interfere with the tissue closure device when carrying the suture into the tissue, which may lead to problems such as inaccurate final arrival position of the access needle, separation of the suture from the access needle, and failure of the suture to be accurately delivered to a predetermined position. Moreover, the feature position for loading a suture in a conventional tissue closure device is often narrow in structure, and the operation of loading the suture can be difficult. In addition, when the suture is loaded, the tip of the access needle is usually exposed, which may pose a puncture risk to the operator and may also have certain hygiene hazards.

US 2002/0005204 A1 discloses a suture passer adapted for grasping and passing internal sutures, such as to construct a sling. The suture passer is particularly suited for use in connection with such surgery as bladder suspension procedures disclosed therein, where sutures are required to be advanced and withdrawn without direct visualization and through relatively long distances. The suture passer comprises a handle, an axially movable probe, and a probe guide having a suture channel.

EP 3473189 A1 discloses suturing devices and systems used to apply sutures and/or to close openings at, within, or into a biological structure. The suturing device comprises an elongate member having a proximal end, a distal end, one or more distal arms, one or more proximal extensions, and one or more needles. A sheath may be used with the device to maintain or substantially maintain haemostasis while the device is used and while a procedure is performed in the biological structure.

Therefore, there is a need to provide a tissue closure device and a method of using the same in order to at least partially address the aforementioned problems.

### BRIEF SUMMARY

The present invention provides a tissue closure device as recited in claim 1. Optional features are recited in the dependent claims. An access needle can be preloaded in the tissue closure device, and the tissue closure device and the access needle form an integrated unit which can be delivered to an operator conveniently. In the present invention, the unique arrangement of an access needle and a suture slot of a main body housing enables an operator to conveniently load a suture into the access needle and release the suture from the main body housing, and also facilitates exit of the access needle carrying the suture from the tissue closure device. The entire operating process is fast and the control over the access needle is accurate, thus achieving a better operation effect. In a suture loading process, a tip of the access needle is concealed in the main body housing, so that an operator can be protected from a puncture wound, and the tip of the access needle is less likely to be contaminated.

Also disclosed but not explicitly claimed is a method which can be performed using a tissue closure device according to the present invention. The method comprises:
a suture loading step comprising: loading a suture into a suture carrying portion of an access needle via a suture loading slot of a main body housing; and
a needle exiting step comprising: causing the access needle loaded with the suture to extend outwardly from a needle exit hole of the main body housing to an actuated position.

### BRIEF DESCRIPTION OF DRAWINGS

To better understand the above and other objectives, features, advantages and functions of the present invention, reference may be made to preferred embodiments shown in the accompanying drawings. The same or similar reference numerals refer to the same or similar components in the accompanying drawings. It should be understood by those skilled in the art that the drawings are intended to schematically illustrate the preferred embodiments of the invention and have no limitation on the scope of the invention, and various members are not drawn to scale.
FIG. 1 is a schematic perspective view of a tissue closure device according to a preferred embodiment of the present invention, wherein an access needle is in an initial position and a stabilizer is in a folded position;
FIG. 2 is a partially enlarged schematic diagram of the structure at a suture slot of a main body housing in FIG. 1;
FIG. 3 is a schematic section view of FIG. 2, wherein the exterior structure of the main body housing is partially omitted to show the access needle;
FIG. 4 is a schematic diagram of the structure at the suture slot in FIG. 2 taken from another angle of view;
FIG. 5 is a schematic diagram of the structure at the suture slot in FIG. 2 taken from still another angle of view;
FIG. 6 is a schematic perspective view in which the main body housing in FIG. 1 is omitted;
FIG. 7 is a schematic diagram of the tissue closure device in FIG. 1 in another state, wherein the access needle is in an actuated position and the stabilizer is in an unfolded position;
FIG. 8 is a schematic perspective view in which the main body housing in FIG. 7 is omitted;
FIG. 9 is a schematic diagram showing cooperation of a needle control component and a stabilizer control component in FIG. 8;
FIG. 10 is a schematic section view of FIG. 9, which shows the interior structure of the needle control component;
FIG. 11 is a schematic exploded perspective view of the needle control component, the access needle, and a fixing pin in FIG. 10;
FIG. 12 is a schematic diagram of another embodiment of the present invention, which only shows the structure at a suture slot of a main body housing;
FIG. 13 is a schematic diagram of FIG. 12 taken from another angle of view;
FIG. 14 is a schematic diagram of still another embodiment of the present invention, which only shows the structure at and near a suture slot of a main body housing;
FIG. 15 is a schematic diagram of yet another embodiment of the present invention, which only shows the structure at a suture slot and a suture winding portion of a main body housing;
FIG. 16 is a schematic diagram of an alternative embodiment of FIG. 15, which also only shows the structure at a suture slot and a suture winding portion of a main body housing;
FIGs. 17 and 18 are schematic diagrams of a suture slot portion according to yet another embodiment of the present invention, wherein FIGs. 17 and 18 are taken from different angles of view, and FIG. 18 is a partial view; and
FIG. 19 is a section view of FIG. 18, wherein the structure of a main body housing is partially omitted to show an access needle.

Description of reference numerals:
100 Tissue closure device
10, 610, 710, 810, 910, 1010 Main body housing
11 Housing joining line
12, 1012 Suture slot
121, 6121, 7121, 8121, 9121 Suture loading slot
1211, 10121 First slot portion
1212, 10122 Second slot portion
1212a Flared profile
122, 6122, 7122, 8122, 9122 Suture releasing slot
14 Left half housing
15 Right half housing
16, 616, 716, 816, 916, 1016 Needle exit hole
17 Needle channel
618 Cutting surface
101 First window
102 Second window
20 Needle control component
21 Needle operation portion
22 Needle actuation portion
221 Radially recessed section
222 Access needle mounting hole
223 Fixing pin
2231 Fixing pin hole
224 Position-limiting slot
30 Stabilizer control component
31 Stabilizer operation portion
32 Positioning protrusion
33 Stabilizer actuation portion
40 Stabilizer
41 Shield
42 Soft rubber block
50, 1050 Access needle
51, 1051 Suture carrying portion
52 Tip of access needle
55 Notch
660, 760, 1060 Suture retaining portion
661 Protrusion portion
711 Recessed portion
761 Central portion
762 End portion
811, 911 Suture winding portion
8111 Mounting base portion
8112 Suture winding portion body
8113 Thin neck portion
813, 913 Suture accommodating portion
8131, 9131 Tip of suture accommodating portion
814 Recess structure

### DETAILED DESCRIPTION

A tissue closure device according to the present invention is described in detail below with reference to the accompanying drawings. The embodiments described below are merely preferred embodiments according to the present invention, and on the basis of the preferred embodiments, those skilled in the art can conceive of other modes capable of implementing the present invention, which also fall within the scope of the present invention as defined by the claims.

The present invention provides a tissue closure device for surgical procedures. First of all, it should be noted that the directional terms and position terms mentioned in the present invention should be construed as relative directions and relative positions. For example, "axis direction" and "axial direction" and the like mentioned in the present invention can be construed as the direction along the axis of the main body housing; "radial direction", "circumferential direction" and the like are radial and circumferential directions with respect to the axis; "distal end" and "distal" refer to the direction along the axis and away from the operator (e.g. a surgeon); and "proximal end" and "proximal" refer to the direction along the axis and close to the operator (e.g. a surgeon).

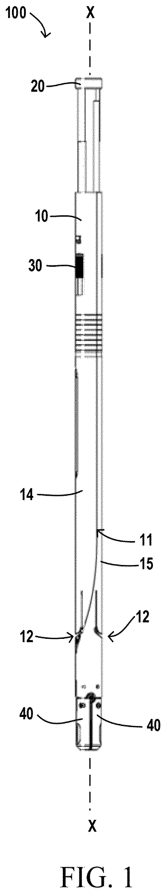

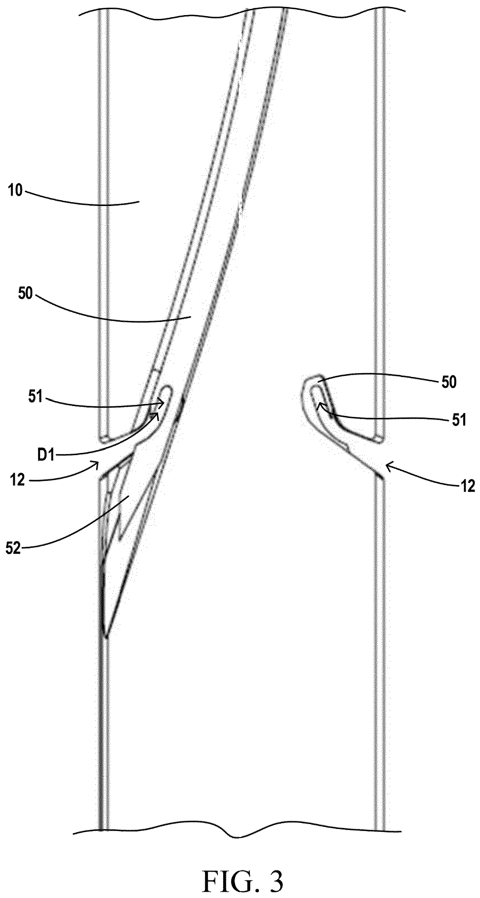

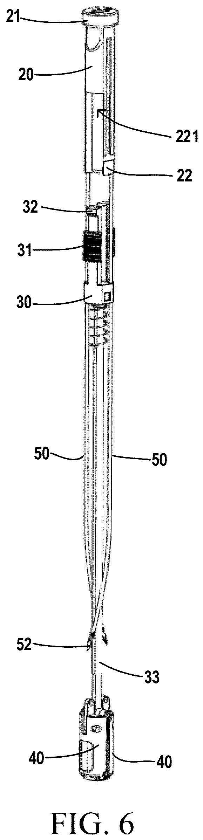

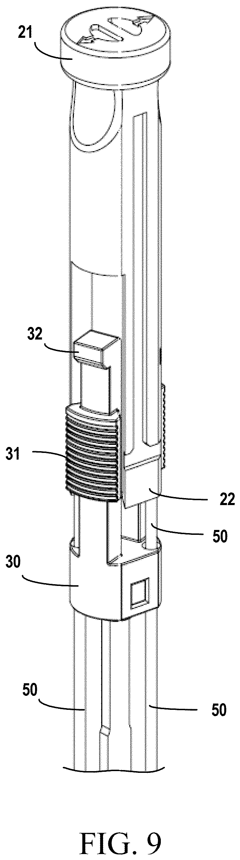

FIGs. 1 to 11 show one preferred embodiment of the present invention. First referring to FIG. 1, a tissue closure device 100 includes a main body housing 10 having a longitudinal axis, and the main body housing 10 internally defines a chamber extending along the longitudinal axis. The chamber is preloaded with an access needle 50 therein (for example, referring to the view in FIG. 6 with the main body housing 10 removed) and has a needle channel (referring to FIG. 3) for the access needle 50 to move. The access needle 50 is used to carry a suture into the tissue of a patient. The tissue closure device 100 further includes a needle control component 20 mounted at a proximal portion thereof, and the needle control component 20 is fixedly connected to a proximal end of the access needle 50. The needle control component 20 can move axially relative to the main body housing 10, thereby controlling the access needle 50 to move from an initial position to an actuated position.

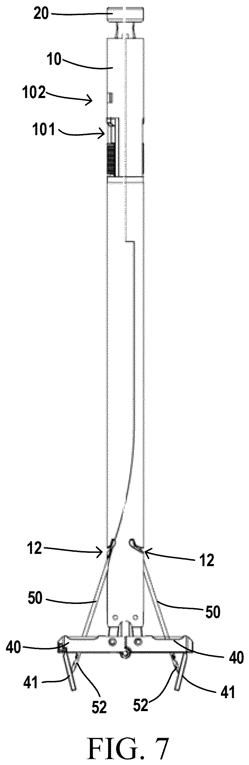

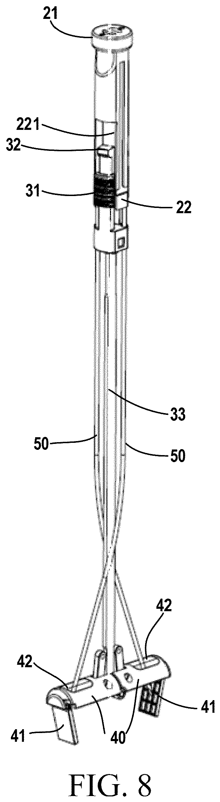

Reference may be made to FIGs. 1 to 6 for the state of the access needle 50 in the initial position, and reference may be made to FIGs. 7 and 8 for the state of the access needle 50 in the actuated position. When the access needle 50 is in the initial position relative to the main body housing 10, the access needle 50 is entirely located in the chamber of the main body housing 10, and a distal end 52 of the access needle 50 is in the most proximal position relative to the main body housing 10. When the access needle 50 is in the actuated position relative to the main body housing 10, the distal end 52 of the access needle 50 is located outside the main body housing 10 and the distal end 52 of the access needle 50 is in the most distal position relative to the main body housing 10.

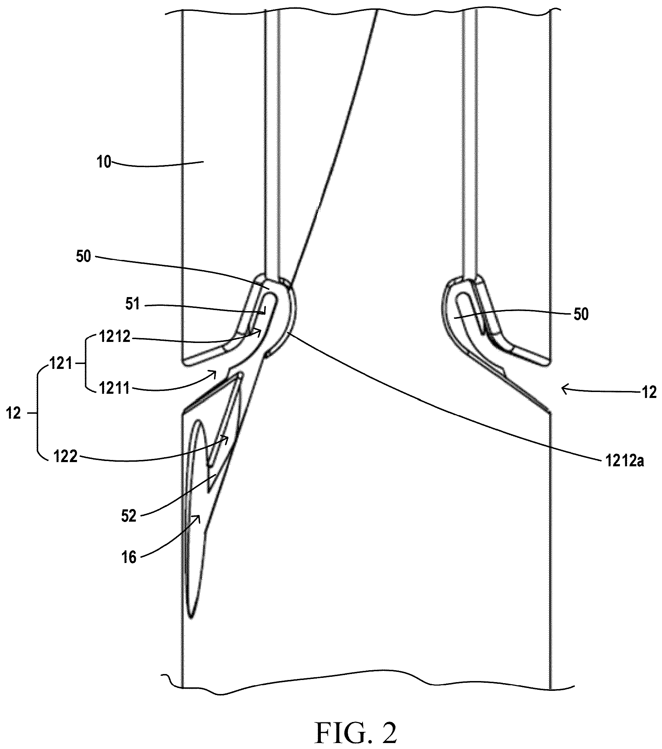

Also referring to FIGs. 2 to 5, a suture slot 12 that communicates the chamber of the main body housing 10 with the outside is provided on a sidewall of the main body housing 10, and the suture slot 12 at least partially extends along the radial direction of the main body housing 10. The access needle 50 is formed with a suture carrying portion 51 close to the distal end 52 thereof, and the suture carrying portion 51 is also in the form of a slot structure extending at least partially proximally along the extending direction of the access needle 50. The needle control component 20 can hold the access needle 50 in the initial position in a predetermined orientation. When the access needle 50 is held in the initial position, the distal end 52 of the access needle 50 is located in the chamber. The "predetermined orientation" means that the orientation of the access needle in the initial position enables the suture slot 12 of the main body housing 10 and the suture carrying portion 51 of the access needle 50 to be at least partially communicated, such that the operator can load a suture into the suture carrying portion 51 of the access needle 50 via the suture slot 12 of the main body housing 10 (it can be appreciated that if the access needle 50 is rotated with respect to the predetermined orientation, the suture carrying portion 51 may not communicate with the slot 12, resulting in failure to successfully load the suture). For example, the inner wall of the suture slot 12 is defined to have a flat surface, such that the access needle 50 is fit to the flat surface and prevented from twisting in the suture slot 12, thus ensuring partial communication between the suture carrying portion 51 and the suture slot 12.

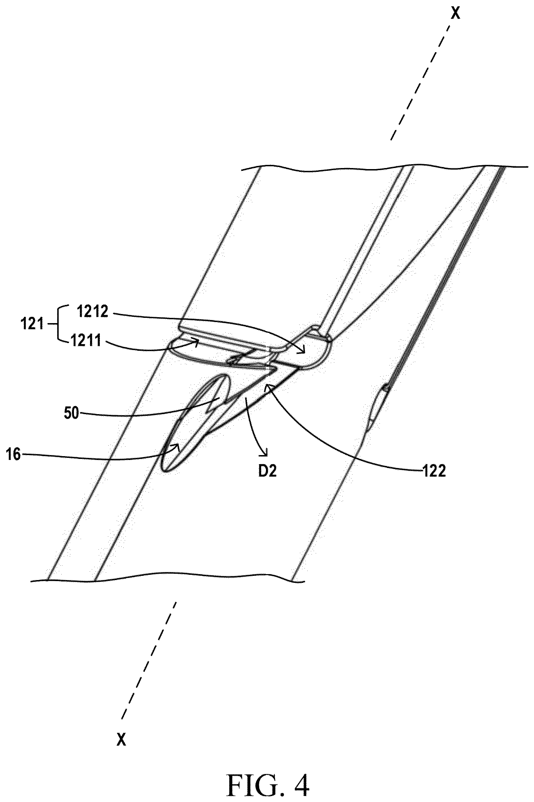

The suture slot 12 may include, for example, a suture loading slot 121 and a suture releasing slot 122. The suture releasing slot 122 may, for example, communicate at a distal end of the suture loading slot 121 and a distal end of the suture releasing slot 122 may further communicate with a needle exit hole 16. The needle control component 20 can control the access needle 50 to extend to an actuated position at a predetermined depth. In the process of the access needle 50 moving from the initial position to the actuated position, the distal end 52 of the access needle 50 will extend through the needle exit hole 16 and exit the main body housing 10. The portion serving as the suture loading slot 121 of the suture slot 12 is used to load a suture into the suture carrying portion 51 from the outside of the tissue closure device 100 when the access needle 50 is held in the initial position in the predetermined orientation, wherein the movement direction of the suture in this process is shown by, for example, an arrow D1 in FIG. 3. The portion serving as the suture releasing slot 122 of the suture slot 12 is used to allow the suture to exit the tissue closure device via the suture releasing slot 122 after the access needle has been moved to the actuated position via the needle exit hole 16, wherein the movement direction of the suture in this process is shown by, for example, an arrow D2 in FIG. 4.

Further, the suture loading slot 121 has a first slot portion 1211 and a second slot portion 1212. The first slot portion 1211 extends at least partially towards the axis from the outside to the second slot portion 1212, and the second slot portion 1212 extends at least partially axially proximally from the first slot portion 1211, wherein the second slot portion 1212 and the suture carrying portion 51 are at least partially aligned. For example, as shown in FIGs. 2 and 3, when the access needle 50 is in the initial position, the extending directions of the second slot portion 1212 and the suture carrying portion 51 of the access needle 50 are substantially the same, as can be seen from a side view of the tissue closure device 100. More specifically, in this embodiment, the first slot portion 1211 extends both radially inward and axially proximally, and the second slot portion 1212 also extends radially inward and axially proximally. An included angle between the extending direction of the second slot portion 1212 and the longitudinal axis is less than an included angle between the first slot portion 1211 and the longitudinal axis.

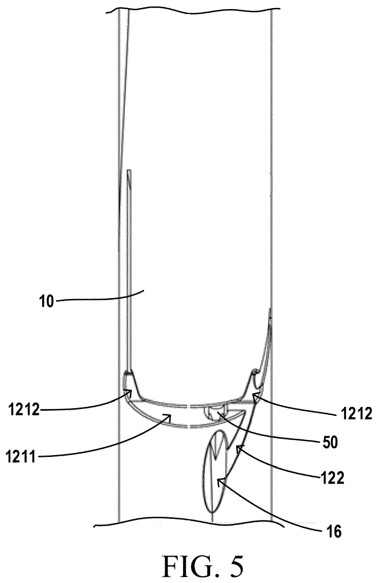

Also referring to FIGs. 4 and 5, the first slot portion 1211 constitutes an elongated opening formed on the sidewall in a direction perpendicular to the longitudinal axis, and two tips of the elongated opening respectively extend proximally and towards the axis to jointly define the second slot portion 1212. The extending directions of the suture releasing slot 122 and the second slot portion 1212 are substantially the same, and the suture releasing slot 122 smoothly transitions from the second slot portion 1212 to the needle exit hole 16. It can be clearly seen from FIGs. 4 and 5 that, the needle exit hole 16 is an opening fitting the shape of the access needle 50, and the opening may have, for example, a round or oval contour.

Because the suture slot 12 is formed on the main body housing 10 made of a rigid material in this embodiment, the suture slot 12 needs to have a certain width to allow the suture to pass through. For example, the width of the suture loading slot 121 is greater than or equal to the width of the suture carrying portion 51 of the access needle 50. Preferably, the suture loading slot 121 may have a clearance design at the boundary, so that after the suture enters the suture loading slot 121, the suture carrying portion 51 can also be pressed to drive the distal end of the access needle 50 to move in the suture slot 12 in a very small range, thus more accurately completing suture loading of the access needle 50. The clearance design may include, for example, a flared profile 1212a relative to the center of the suture loading slot, that is formed on the boundary of the second slot portion 1212 of the suture loading slot 121, and the flared profile 1212a may be, for example, a curved segment. The clearance design may also have other forms.

Referring to FIGs. 1, and 6 to 8 as a whole, in this embodiment, two access needles 50 are provided, and the proximal ends of the two access needles 50 are fixed on the same needle control component 20, so that the needle control component 20 controls the two access needles 50 simultaneously. The access needle 50 is easily bendable and flexible, and is made of, for example, a plastic material. The chamber in the main body housing 20 includes a needle channel which is configured to enable the access needle 50 to move therein in a predetermined pose. In other words, the form of the needle channel determines the form of the access needle 50. It can be seen from FIGs. 6 and 8 that, in the initial position, each access needle 50 may include, for example, a straight segment and a curved segment located at the distal end of the straight segment, and the curved segment extends distally partially about the longitudinal axis. Then, it can be understood that, in fact, it is the needle channel that has such straight and curved segments. In the actuated position, the access needle 50 may include two straight segments and a curved segment between the two straight segments. The curved segment extends partially about the longitudinal axis, the straight segment located proximally of the curved segment extends axially, and the straight segment located distally of the curved segment extends both axially and radially. Likewise, it can be understood that, in fact, it is the needle channel that has such straight and curved segments (certainly, except for the portions outside the main body housing).

Also referring to FIG. 8, under the operation of the needle control component 20 and the position-limiting effect of the needle channel, when the distal ends 52 of the access needles 50 reach the actuated position, a connecting line between the distal ends 52 of the two access needles 50 is in a radial direction with respect to the axis, and the radial direction is, for example, also the extending direction of a stabilizer 40 (which will be described in detail later).

The two needle channels are symmetrical about the axis at any axial position. As shown in FIGS. 6 and 8, such arrangement enables the portions of the two access needles 50 at any axial position to be symmetrical about the longitudinal axis. Correspondingly, two perforated structures are correspondingly provided on the sidewall of the main body housing 10, and each of the perforated structures includes a suture slot 12 and a needle exit hole 16. The two perforated structures are substantially symmetrical about the axis. For example, the corresponding parts of the two perforated structures are symmetrical about the axis. Further, for example, the respective suture loading slots 121 of the two perforated structures are symmetrical about the axis, the respective suture releasing slots 122 are symmetrical about the axis, and the respective needle exit holes 16 are symmetrical about the axis.

Referring to FIGs. 1, 7 and 8, the tissue closure device 100 further includes a stabilizer 40, and a stabilizer control component 30 mounted on the main body housing 10 close to a proximal end thereof. The stabilizer 40 includes, for example, at least two wing portions that are located on a distal end of the main body housing 10. The stabilizer control component 30 can move axially with respect to the main body housing 10 in response to operation of an operator, thereby driving the at least two wing portions to be actuated between a folded position and an unfolded position. For example, when the stabilizer control component 30 moves distally relative to the main body housing 10, the stabilizer 40 is actuated from the folded position to the unfolded position. In the state shown in FIG. 1, the stabilizer 40 is in the folded position. The stabilizer 40 in the folded position extends substantially axially distally from the main body housing 10, and the radial dimension of the stabilizer 40 in the folded position is approximately equal to the outer diameter of the main body housing 10. In the states shown in FIGs. 7 and 8, the stabilizer 40 is in the unfolded position, and the extending direction of the stabilizer 40 in the unfolded position is substantially perpendicular to the longitudinal axis. The stabilizer 40 may be used as, for example, a depth limiting mechanism. When the distal end of the tissue closure device 100 extends to approximately a predetermined depth within the patient, the stabilizer control component 30 may be operated to unfold the stabilizer 40. The proximal surface of the unfolded stabilizer 40 is fit to the inner wall of the tissue of the patient, thereby controlling the extension of the tissue closure device 100 to the current depth within the patent.

A soft rubber block 42 may be disposed on the stabilizer 40. In the process of the access needle 50 moving from the initial position to the actuated position, the distal end 52 of the access needle 50 will pass through the soft rubber block 42 and reach the distal position of the stabilizer 40. Preferably, in order to prevent the distal end 52 of the access needle 50 from piercing the tissue of the patient, a shield 41 extending distally from the stabilizer 40 may be provided at a radial outer side of the stabilizer 40. When the access needle 50 is in the actuated position, the shield 41 provides protection at a radial outer side of the distal end 52 of the access needle 50. Additionally or alternatively to the above preferred arrangements, this embodiment may also have preferred arrangements in some aspects. The preferred arrangements will be discussed in detail below with reference to FIGs. 1, and 7 to 11.

For example, this embodiment may have some preferred arrangements in terms of the form of the needle control component 20, the joining manner between the needle control component 20 and the main body housing 10, and the connection manner between the needle control component 20 and the access needle 50. The needle control component 20 has a needle operation portion 21 located outside the main body housing 10 for a user to operate, and a needle actuation portion 22 located in the chamber and connected to the access needle 50. The chamber of the main body housing 10 may have a proximal opening from which the needle operation portion 21 extends to form a button structure for the operator to press. Further, a position-limiting structure is provided at the connection position between the needle control component 20 and the main body housing 10, and the position-limiting structure only allows the needle control component 20 to move axially relative to the main body housing 10 and prevents the needle control component 20 from rotating or translating in other directions relative to the main body housing 10.

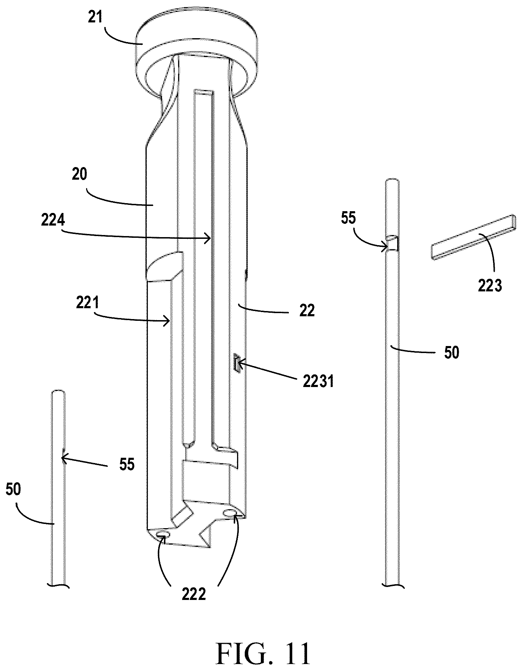

The position-limiting structure includes, for example, an axially extending position-limiting slot 224 (as shown in FIG. 11) located on an outer surface of the actuation portion, and a protrusion located on an inner surface of the main body housing 10 and having a width matching that of the position-limiting slot 224. In this embodiment, the main body housing 10 includes a left half housing 14 and a right half housing 15, and the left half housing 14 and the right half housing 15 are butted together along a direction transverse to the longitudinal axis, with a housing joining line 11 formed at the connection position therebetween. The left half housing 14 and the right half housing 15 are respectively provided with protruding halves at portions thereof adjacent to each other. The protruding halves of the left half housing 14 and the right half housing 15 are butted together to jointly define the protrusion.

It can be understood that, in this embodiment, the protrusion can slide along the position-limiting slot 224 so that the needle control component 20 moves from a first position (for example, a proximal position) to a second position (for example, a distal position) relative to the main body housing 10. When the needle control component 20 is located at the first position, the needle control component 20 holds the access needle 50 in the initial position in a predetermined orientation. When the needle control component 20 moves from the first position to the second position, the access needle 50 is driven to move from the initial position to the actuated position at a predetermined depth.

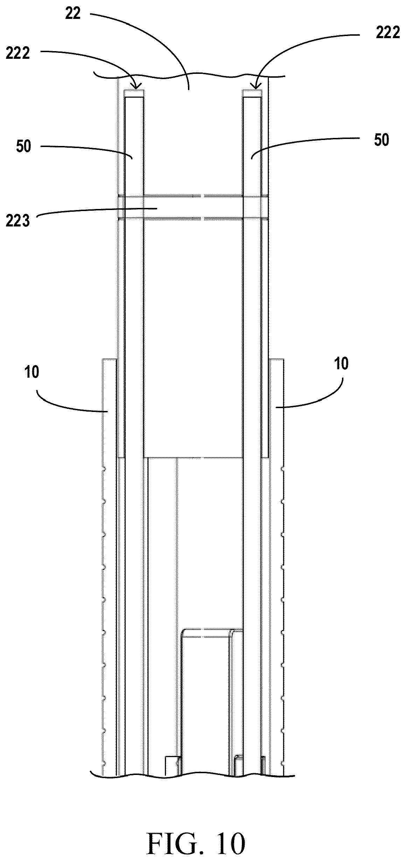

Referring to FIGs. 10 and 11, the needle actuation portion 22 has two holes 222 that are open distally and extend axially, and the inner diameter of each hole 222 matches the outer diameter of one access needle 50. And a fixing pin 223 transverse to the longitudinal axis is provided in the needle control component 20 and the fixing pin 223 is connected between the two holes 222. Each access needle 50 may be provided with a notch 55 for receiving the fixing pin 223, and the fixing pin 223 is engaged and fixed at the notch 55. That is to say, one fixing pin 223 is joined to two access needles 50 simultaneously, so that the two access needles 50 are fixed to the needle actuation portion 22 simultaneously. The needle actuation portion 22 is provided with a fixing pin hole 2231 for insertion of the fixing pin 223 in an assembly stage.

The wall surface of the notch 55 may include a flat surface, so as to enhance the joining stability between the fixing pin 223 and the access needle 50. The notch 55 may extend in the middle portion of the access needle 50, and may also extend all the way to the proximal tip of the access needle 50. It should be noted that, the notch is only an example of the "mating portion", and the mating portion may also have other forms in other embodiments that are not shown. In some embodiments, the fixing pin may not be provided, and the mating portion is directly molded together with the needle control component.

This embodiment may also have some preferred arrangements in terms of the connection manner between the stabilizer control component 30 and the main body housing 10, and the connection manner between the stabilizer control component 30 and the needle control component 20. For example, the sidewall of the main body housing 10 is provided with a first window 101 and a second window 102 located proximally of the first window 101 (referring to FIG. 7). The stabilizer control component 30 includes a stabilizer operation portion 31 exposed through the windows, a positioning protrusion 32 located proximally of the stabilizer operation portion 31 and fixed relative to the stabilizer operation portion 31, and a stabilizer actuation portion 33 located distally of the stabilizer operation portion 31. A radial outer portion of the stabilizer operation portion 31 has an axial dimension smaller than that of the first window 101 so as to be able to axially slide in the first window 101. The size of a radial outer portion of the positioning protrusion 32 matches that of the second window 102. When the positioning protrusion 32 is placed in the second window 102, the stabilizer control component 30 is locked relative to the main body housing 10, for example, as shown in FIG. 1. When the positioning protrusion 32 is located in the second window 102, the operator is allowed to press the positioning protrusion 32 radially inwardly, and the positioning protrusion 32 is biased inwardly to disengage from the second window 102 to unlock the stabilizer control component. Subsequently, the positioning protrusion 32 can be actuated distally together with the stabilizer operation portion 31. When the stabilizer control component 30 actuates the stabilizer 40 to the unfolded position, the positioning protrusion 32 is positioned in the first window 101 together with the stabilizer operation portion 31, as shown in FIGs. 7 and 8. Preferably, the axial dimension between the most proximal end of the positioning protrusion 32 and the most distal end of the stabilizer operation portion 31 is just equal to the axial dimension of the first widow 101, such that the stabilizer control component 30 cannot move axially relative to the main body housing 10 when the positioning protrusion 32 is located in the first window 101 together with the stabilizer operation portion 31.

Further, there are two stabilizer operation portions 31. The stabilizer control component 30 further includes a stabilizer actuation portion 33 fixedly connected at the distal ends of the two stabilizer operation portions 31. A radially recessed section 221 (as shown in FIG. 11) is provided on a sidewall of the needle control component 20, and at least a radial inner portion of the stabilizer operation portion 31 is correspondingly mounted at the radially recessed section 221. The needle control component 20 is partially located between the two stabilizer operation portions 31 and is completely located proximally of the stabilizer actuation portion 33.

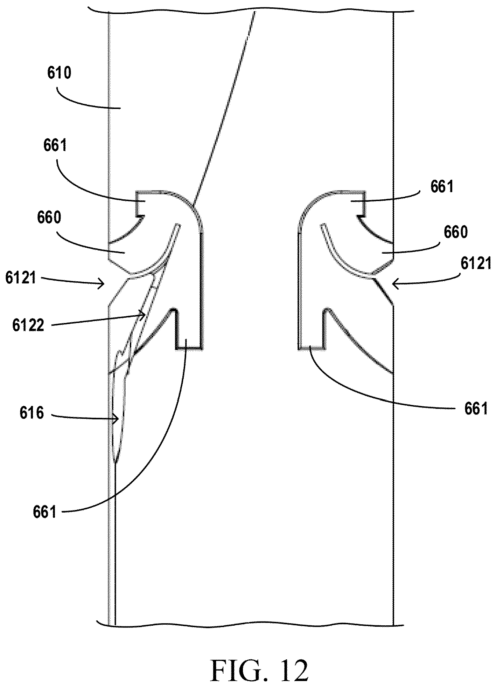

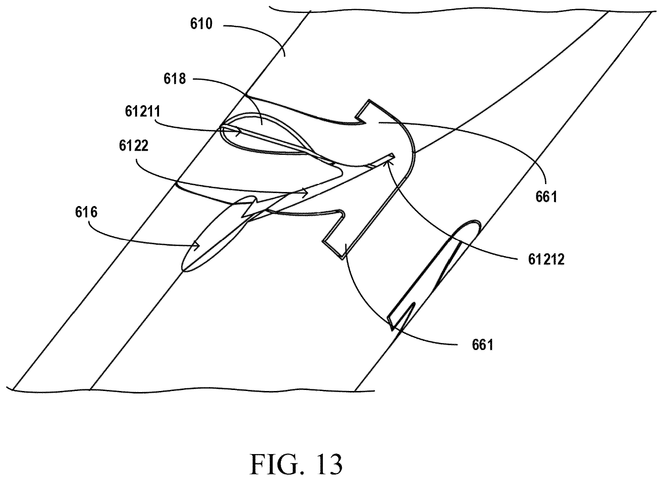

FIGs. 12 and 13 show the structure at the suture slot of the tissue closure device according to another preferred embodiment of the present invention. A main body housing 610 includes a rigid sidewall and a suture retaining portion 660 that can provide greater friction. The suture retaining portion 660 is made of, for example, a flexible, plastic, or elastic material. In an embodiment, the suture retaining portion 660 may be made of a soft rubber. The suture retaining portion 660 can hold the suture carried by the access needle in place by means of friction when the access needle is in the initial position. For example, a window is formed on the rigid sidewall, and the suture retaining portion 660 is mounted at the window and constitutes a part of the sidewall of the main body housing 610, wherein at least a part of the suture slot is formed on the suture retaining portion 660.

As shown in FIGs. 12 and 13, a suture loading slot 6121 and a suture releasing slot 6122 are both formed on the suture retaining portion 660, and a needle exit hole 6122 is jointly defined by the suture retaining portion 660 and the rigid sidewall. In this embodiment, the suture loading slot 6121 also includes a first slot portion 61211 and a second slot portion 61212, and cutting surfaces 618 that function as a guide structure are formed at an inlet of the first slot portion 61211. The cutting surfaces 618 are provided proximally and distally of the inlet respectively, to facilitate loading of the suture from the outside via the suture slot into the suture carrying portion of the access needle.

In this embodiment, because the suture retaining portion 660 can provide friction to the suture, a suture slot 612 formed on the suture retaining portion 660 can have a smaller width. For example, the width of the suture slot 612 can be smaller than the width of the suture slot in the embodiment shown in FIGs. 1 to 11. Further, the width of the suture slot 612 can be even smaller than or equal to the width of the suture, such that when the suture enters the suture slot 612, an interference fit is created between the suture and the suture slot 612, and a slight deformation may occur at the suture slot 612. This slight deformation does not affect the loading of the suture, but can help to clamp the suture, avoiding the suture from coming off. It can be understood that, if the suture slot is partially defined by the suture retaining portion (for example, the suture is jointly defined by the suture retaining portion and the rigid sidewall), the suture slot can still have such a smaller width.

Preferably, the suture retaining portion 660 is provided with a protrusion portion 661 protruding along the sidewall of the main body housing 610, a corresponding recess portion is provided at the window of the rigid sidewall, and the protrusion portion 661 and the recess portion cooperate to secure the suture retaining portion 660 and the rigid sidewall. Additionally or alternatively to this solution, a protrusion portion protruding along the sidewall of the main body housing is provided at the window of the rigid sidewall, a corresponding recess portion is provided at the edge of the suture retaining portion, and the protrusion portion and the recess portion cooperate to secure the suture retaining portion and the rigid sidewall.

It should be noted that, in addition to the foregoing structures, this embodiment is identical to or similar to the embodiment shown in FIGs. 1 to 11, and the description of the embodiment in FIGs. 1 to 11 should also be regarded as description of this embodiment.

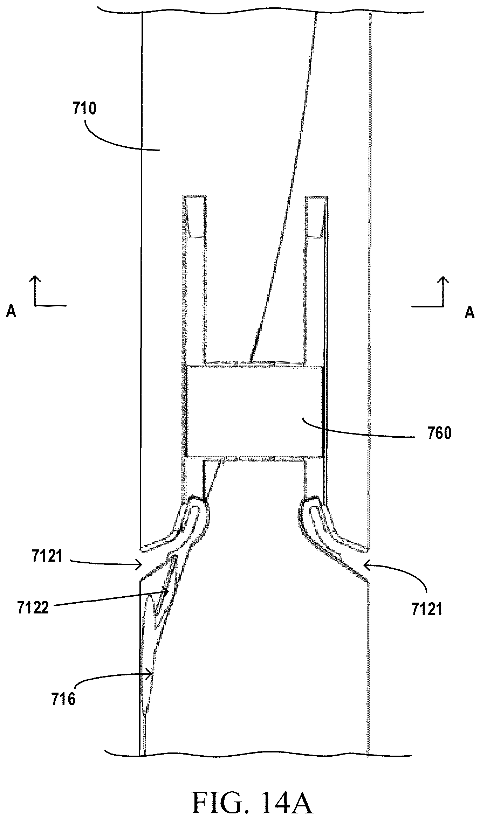

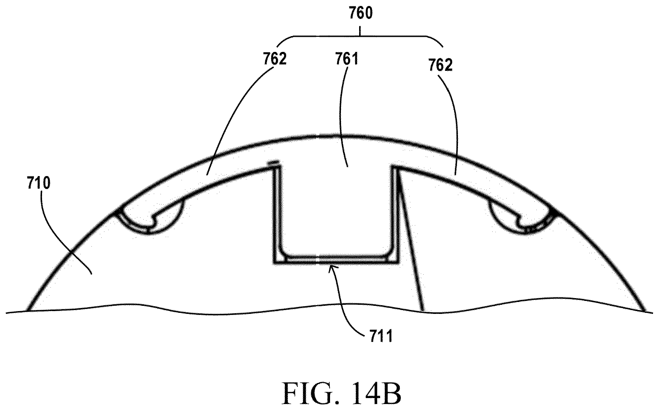

FIGs. 14A and 14B show still another preferred embodiment of the present invention. In this embodiment, a suture retaining portion 760 is different from the suture retaining portion in FIGs. 12 and 13. In the embodiment shown in FIG. 14A, a suture loading slot 7121, a suture releasing slot 7122, and a needle exit hole 716 are provided on the sidewall of the main body housing 710, and the structural forms of the suture loading slot 7121, the suture releasing slot 7122, and the needle exit hole 716 are identical to or similar to those in FIGs. 1 to 11. In FIG. 14, the suture retaining portion 760 is mounted on the rigid sidewall and independent of the suture loading slot 7121, the suture releasing slot 7122, and the needle exit hole 716. The suture retaining portion 760 allows the suture to enter between the suture retaining portion 760 and the rigid sidewall so as to clamp the suture between the suture retaining portion 760 and the rigid sidewall. Preferably, the suture retaining portion 760 may be mounted proximally of the suture loading slot 7121, and located between the two suture loading slots 7121 circumferentially.

Preferably, the outer surface of the suture retaining portion 760 is flush with the outer surface of the rigid sidewall. It can be understood that, in order to achieve this feature, a recessed portion 711 will be provided on the rigid sidewall, and the suture retaining portion 760 is inserted into the recessed portion with the shape thereof fit to the same. More preferably, referring to FIG. 14B, the suture retaining portion includes a central portion 761 and end portions 762 located at both circumferential ends of the central portion 761, and the inner surface of the central portion 761 protrudes further inwardly toward the longitudinal axis relative to the inner surfaces of the end portions 762.

It should be noted that, in addition to the foregoing structures, this embodiment is identical to or similar to the embodiment shown in FIGs. 1 to 11, and the description of the embodiment in FIGs. 1 to 11 should also be regarded as description of this embodiment.

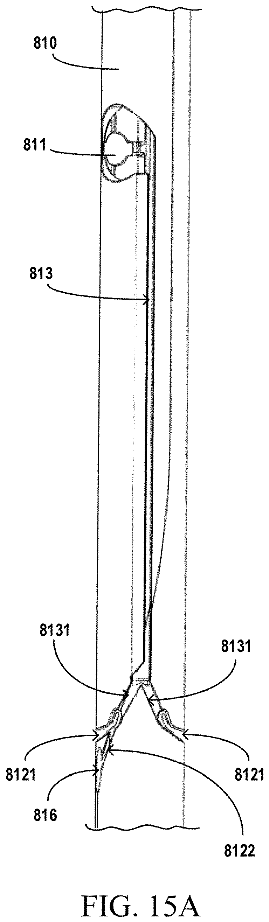

FIGs. 15A to 16 show some other preferred embodiments of the present invention, and mainly illustrate two forms of a suture winding portion provided on a main body sidewall. It should be noted that, the suture winding portion in FIGs. 15A to 16 may be applied to the embodiments in FIGs. 1 to 14.

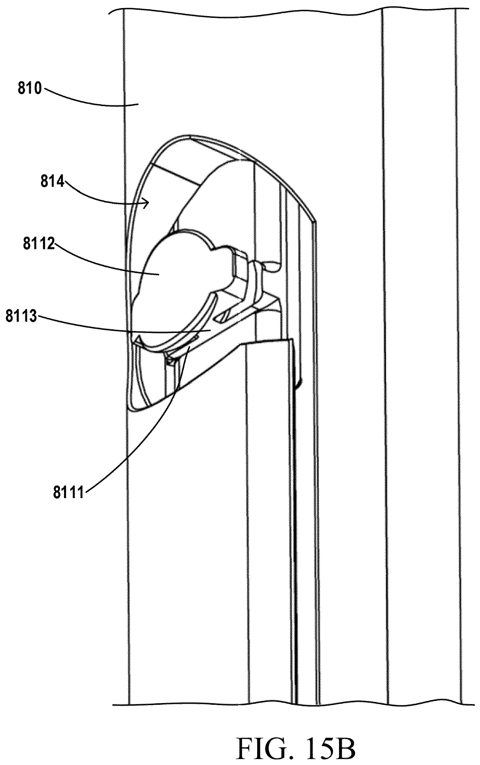

Referring to FIG. 15A, a suture loading slot 8121, a suture releasing slot 8122, and a needle exit hole 816 that are similar to those in the embodiment of FIGs. 1 to 11 are provided on the sidewall of a main body housing 810. The main body housing is further provided with a recessed and externally open suture accommodating portion 813 that extends parallel to the longitudinal axis, the distal end of the suture accommodating portion 813 communicates with the suture loading slot 8121, and the proximal end of the suture accommodating portion 813 is provided with a suture winding portion 811 for loading the suture. Two branches 8131 are formed at the distal end of the suture accommodating portion 813, so as to communicate with the two suture loading slots 8121, respectively. It can be seen from FIG. 15B that, the suture winding portion 811 is mounted in a recess structure 814 on the main body housing 810, and the suture winding portion 811 is formed into a round button and includes a mounting base portion 8111 directly joined to the main body housing 810 and a suture winding portion body 8112 located on the outer side of the mounting base portion 8111. The mounting base portion 8111 and the suture winding portion body 8112 are connected by a thin neck portion 8113, and the suture is wound around the thin neck portion 8113.

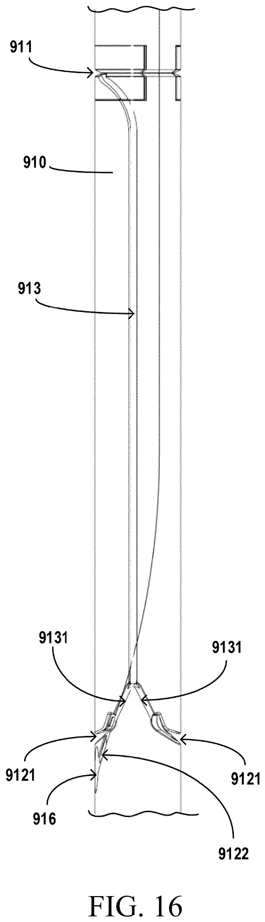

Referring to FIG. 16, a suture loading slot 9121, a suture releasing slot 9122, and a needle exit hole 916 that are similar to those in the embodiment of FIGs. 1 to 11 are provided on a main body housing 910. The main body housing 910 is further provided with a recessed and externally open suture accommodating portion 913 that extends parallel to the longitudinal axis, the distal end of the suture accommodating portion 913 communicates with the suture loading slot 9121, and the proximal end of the suture accommodating portion 913 is provided with a suture winding portion 911 for loading the suture. Two branches 9131 are formed at the distal end of the suture accommodating portion 913, so as to communicate with the two suture loading slots 9121, respectively. The suture winding portion 911 may be a recessed groove extending along the entire circumference.

It should be noted that, various arrangements in this embodiment may be identical to or similar to the embodiment shown in FIGs. 1 to 11, and the description of the embodiment in FIGs. 1 to 11 should also be regarded as description of this embodiment.

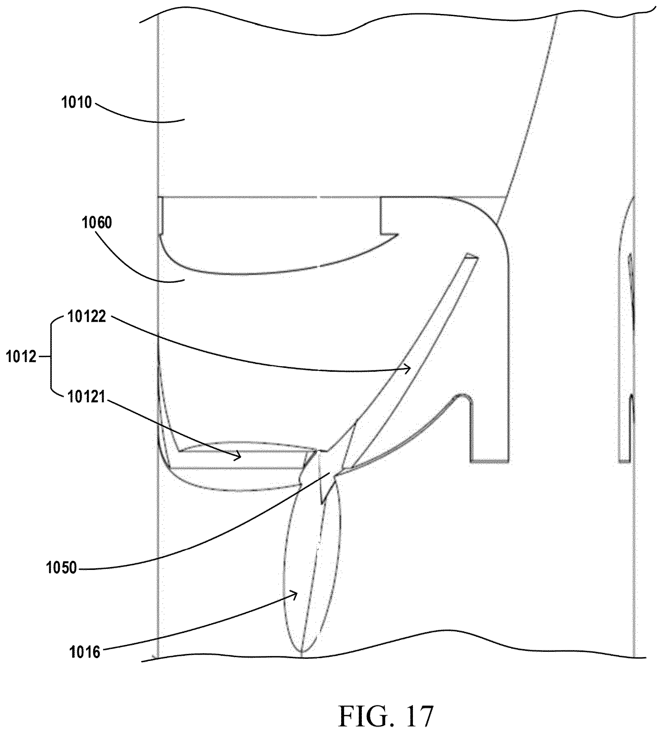

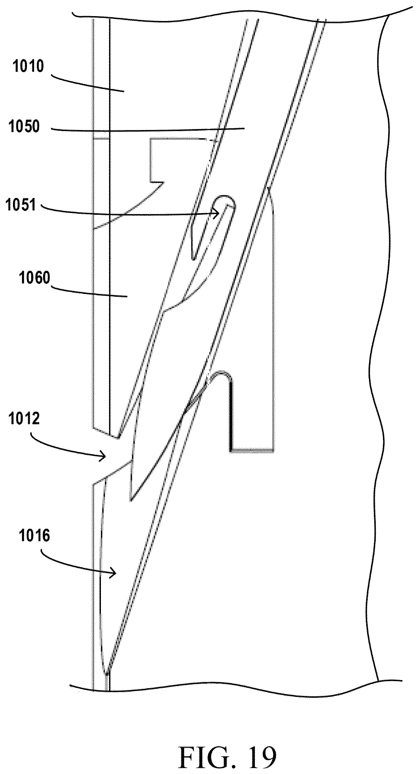

FIGs. 17 to 19 show still another embodiment of the present invention. In this embodiment, the form of the suture slot on the main body sidewall is different from that shown in FIGs. 1 to 16. Referring to FIGs. 17 to 19, a suture slot 1012 is an integrated slot serving as both a suture loading slot and a suture releasing slot. The suture slot 1012 is configured to allow a suture to be loaded from the outside of the tissue closure device via the suture slot 1012 into a suture carrying portion 1051 of an access needle 1050 and to allow the suture to disengage from the tissue closure device via the suture slot 1012. The suture slot 1012 directly communicates with a needle exit hole 1016. Further, the suture slot 1012 is formed on a suture retaining portion 1060 on the main body sidewall, and the needle exit hole 1016 is jointly defined by the suture retaining portion 1060 and the rigid sidewall. The suture slot 1012 also includes a first slot portion 10121 serving as a suture loading inlet, and a second slot portion 10122 extending proximally from the first slot portion 10121 and towards the axis. When a suture is loaded, the suture needs to move proximally along the second slot portion 10122, till the suture reaches the suture carrying portion 1051.

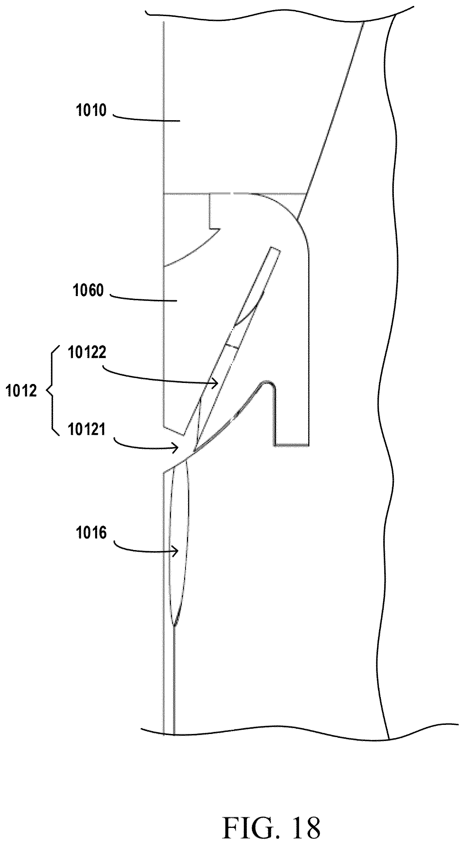

The suture slot includes a first slot portion and a second slot portion. The first slot portion extends at least partially towards the axis from the outside to the second slot portion, and the second slot portion extends at least partially axially proximally from the first slot portion. In the view shown in FIG. 18, the extending length of the second slot portion is several times of the extending length of the first slot portion.

Referring back to FIG. 17, the first slot portion constitutes an elongated opening formed on the sidewall in a direction perpendicular to the longitudinal axis, and two tips of the elongated opening respectively extend proximally and towards the axis to jointly define the second slot portion.

The suture carrying portion of the access needle is concealed in the second slot portion. When a suture is to be loaded, the suture needs to move proximally to the suture carrying portion along the second slot portion. The distal end of the access needle carrying the suture can exit the main body housing via the needle exit hole. In the final step where the suture needs to be released, the suture also exits the main body housing via the integrated suture slot.

It should be noted that, except for the arrangement of the suture slot, various arrangements in this embodiment may be identical to or similar to those in the embodiment shown in FIGs. 1 to 11, and the description of the embodiment in FIGs. 1 to 11 should also be regarded as description of this embodiment.

It should be further noted that, specific arrangements in the foregoing embodiments may be combined to obtain new embodiments, and these new embodiments may also fall within the scope of the present invention as defined by the claims.

Also disclosed is a method of using the tissue closure device in any one of the foregoing solutions. The method includes a suture loading step and a needle exiting step. The suture loading step includes: loading a suture into a suture carrying portion of an access needle via a suture loading slot of a main body housing. The needle exiting step includes: causing a needle control firmware to drive the access needle loaded with the suture to extend outwardly via a needle exit hole of the main body housing to an actuated position, and this step is implemented by, for example, a needle control component.

From the above discussion, it can be seen that the needle control component controls the access needle in two main aspects: control of the orientation of the access needle in an initial position, which enables at least partial communication between the suture carrying portion of the access needle and the suture slot to allow loading of the suture into the suture carrying portion via the suture slot; and control of the actuation depth and the orientation of the access needle in a moving process from the initial position to the actuated position, which enables the access needle to accurately reach the actuated position at a predetermined depth and radial position.

A tissue closure device and an access needle of the present invention form an integrated unit which can be delivered to an operator conveniently. In the present invention, the unique arrangement of an access needle and a suture slot of a main body housing enables an operator to conveniently load a suture into the access needle and release the suture from the main body housing, and also facilitates exit of the access needle carrying the suture from the tissue closure device. The entire operating process is fast and the control over the access needle is accurate, thus achieving a better operation effect. In a suture loading process, the tip of the access needle is concealed in the main body housing, so that an operator can be protected from a puncture wound, and the tip of the access needle is less likely to be contaminated.

From the above, those skilled in the art will readily recognize that alternative structures of the structures disclosed in the present invention may be used as feasible alternative embodiments, and the embodiments disclosed in the present invention may be combined to form new embodiments to the extent that they fall within the scope of the appended claims.

## Claims

1. A tissue closure device (100) comprising:
a main body housing (10; 610; 710; 810; 910; 1010) having a longitudinal axis, the main body housing (10; 610; 710; 810; 910; 1010) internally defining a chamber extending along the longitudinal axis, a suture slot (12) being provided on a sidewall of the main body housing (10; 610; 710; 810; 910; 1010), and the main body defining a needle exit hole (16; 616; 716; 816; 916; 1016); and
an access needle (50; 1050), which is capable of being preloaded in the chamber, is provided with a suture carrying portion (51) close to a tip (52) thereof, is capable of being held in an initial position, and is capable of being actuated distally from the initial position to an actuated position,
wherein when the access needle (50; 1050) is in the initial position, the access needle (50) is accommodated in the chamber, and the suture carrying portion (51) and the suture slot (12) are at least partially communicated to enable a suture to enter the suture carrying portion (51) via the suture slot (12); and when the access needle (50) is in the actuated position, the access needle (50) penetrates through the needle exit hole (16; 616; 716; 816; 916; 1016), and the tip (52) of the access needle (50) is located outside the chamber;
wherein the suture slot (12) comprises a suture loading slot (121; 6121; 7121; 8121; 9121; 10121), the suture loading slot (121; 6121; 7121; 8121; 9121; 10121) being configured to allow the suture to be loaded from the outside of the tissue closure device into the suture carrying portion (51) of the access needle (50) via the suture loading slot (121; 6121; 7121; 8121; 9121; 10121); and **characterized in that**:
the needle exit hole (16; 616; 716; 816; 916; 1016) is provided on the sidewall of the main body housing (10; 610; 710; 810; 910; 1010); and
the suture slot (12) further comprises a suture releasing slot (122; 6122; 7122; 8122; 9122; 10122), the suture releasing slot (122; 6122; 7122; 8122; 9122; 10122) being communicated between the suture loading slot (121; 6121; 7121; 8121; 9121; 10121) and the needle exit hole (16; 616; 716; 816; 916; 1016) and configured to allow the suture to disengage from the tissue closure device (100) via the suture releasing slot (122; 6122; 7122; 8122; 9122; 10122), wherein the suture loading slot (121; 6121; 7121; 8121; 9121; 10121) and the suture carrying portion (51) are at least partially aligned when the access needle (50) is in the initial position.

2. The tissue closure device according to claim 1, wherein the suture slot (1012) is an integrated slot structure serving as both a suture loading slot (10121) and a suture releasing slot (10122), the suture slot (1012) being configured to allow the suture to be loaded from the outside of the tissue closure device into the suture carrying portion of the access needle (1050) via the suture slot (1012) and to allow the suture to disengage from the tissue closure device via the suture slot (1012).

3. The tissue closure device according to claim 1, wherein the suture loading slot (121; 6121; 7121; 8121; 9121; 10121) has a first slot portion (1211; 61211) and a second slot portion (1212; 61212), the first slot portion (1211; 61211) extending at least partially towards the axis from the outside to the second slot portion (1212; 61212), and the second slot portion (1212; 61212) extending at least partially axially from the first slot portion (1211; 61211) along a direction away from the needle exit hole (16; 616; 716; 816; 916; 1016), wherein the second slot portion (1212; 61212) and the suture carrying portion (51) are at least partially aligned; optionally wherein either:
the first slot portion (1211; 61211) constitutes an elongated opening along a direction at least partially perpendicular to the longitudinal axis, two tips of the elongated opening respectively extending along a direction away from the needle exit hole (16; 616; 716; 816; 916; 1016) and towards the axis to jointly define the second slot portion (1212; 61212); or:
the extending directions of the suture releasing slot (122; 6122; 7122; 8122; 9122; 10122) and the second slot portion (1212; 61212) are the same.

4. The tissue closure device according to claim 1, wherein a cutting surface (618) that constitutes a guide structure is provided at an inlet of the slot (6121) on the sidewall of the main body housing (610).

5. The tissue closure device according to claim 1, wherein the needle exit hole (16; 616; 716; 816; 916; 1016) is an opening fitting the shape of the access needle (50).

6. The tissue closure device according to claim 1, wherein two perforated structures that are symmetrical about the longitudinal axis are provided on the sidewall of the main body housing (10), each of the perforated structures comprising at least one said suture slot (12) and at least one said needle exit hole (16; 616; 716; 816; 916; 1016).

7. The tissue closure device according to any one of claims 1-6, wherein the main body housing (610; 710) comprises a non-rigid suture retaining portion (660; 760) configured to be capable of contacting with the suture and holding the suture in place by friction.

8. The tissue closure device according to claim 7, wherein the main body housing (610) comprises a rigid sidewall having a window formed thereon, the suture retaining portion (660) being mounted at the window and constituting a part of the sidewall of the main body housing (610), wherein at least a part of the suture slot is at least formed on the suture retaining portion (660).

9. The tissue closure device according to claim 8, wherein the suture slot comprises a suture loading slot (6121), and a suture releasing slot (6122) communicated between the suture loading slot (6121) and the needle exit hole (616), wherein:
at least a part of the suture loading slot (6121) is formed on the suture retaining portion (660); and/or
at least a part of the suture releasing slot (6122) is formed on the suture retaining portion (660).

10. The tissue closure device according to claim 8, wherein either:
a protrusion portion (661) protruding along the sidewall of the main body housing (610) is disposed on the suture retaining portion (660), a corresponding recess portion is provided at the window of the rigid sidewall, and the protrusion portion (661) and the recess portion cooperate to secure the suture retaining portion (660) and the rigid sidewall; or
a protrusion portion protruding along the sidewall of the main body housing is provided at the window of the rigid sidewall, a corresponding recess portion is provided at the edge of the suture retaining portion, and the protrusion portion and the recess portion cooperate to secure the suture retaining portion and the rigid sidewall.

11. The tissue closure device according to claim 8, wherein a portion serving as a suture loading slot (6121) of the suture slot (612) is at least partially formed on the suture retaining portion (660), and has a width less than or equal to the width of the suture.

12. The tissue closure device according to claim 7, wherein the main body housing (710) comprises a rigid sidewall, the suture retaining portion (760) is mounted on the rigid sidewall and is independent of the suture slot (712), and the suture retaining portion (760) is configured to allow the suture to enter between the suture retaining portion (760) and the rigid sidewall so as to clamp the suture between the suture retaining portion (760) and the rigid sidewall.

13. The tissue closure device according to claim 12, wherein the suture retaining portion (760) is mounted on the side of the suture loading slot (7121) away from the needle exit hole (716); and optionally wherein
two suture slot structures are provided on the sidewall of the main body housing (710), each of the suture slot structures comprises at least one said suture slot, and the suture retaining portion (760) is located between the two suture slot structures.

14. The tissue closure device according to claim 12, wherein a recessed portion (711) is provided on the rigid sidewall, the suture retaining portion (760) is inserted into the recessed portion (711) with the shape thereof fit to the same, and the outer surface of the suture retaining portion (760) is flush with the outer surface of the rigid sidewall; and optionally wherein:
the suture retaining portion (760) comprises a central portion (761) and end portions (762) located at both ends of the central portion (761), the inner surface of the central portion (761) protruding further inwardly toward the longitudinal axis relative to the inner surfaces of the end portions (762).

15. The tissue closure device according to any one of claims 1-6, wherein the suture slot (12) is formed on the rigid sidewall of the main body housing (10), and the portion serving as the suture loading slot (121; 7121; 8121; 9121; 10121) of the suture slot (12) has a width greater than or equal to the width of the suture carrying portion (51) of the access needle (50).

16. The tissue closure device according to claim 17, wherein the boundary of the suture loading slot (121) has a flared profile (1212a) relative to the center of the suture loading slot (121); and optionally wherein
the flared profile (1212a) is a curved profile.

17. The tissue closure device according to any one of claims 1-6, wherein a recessed and externally open suture accommodating portion (813; 913) that extends parallel to the longitudinal axis is provided on the sidewall of the main body housing (810; 910), the suture accommodating portion (813; 913) being connected between the suture slot (8121; 9121) and a suture winding portion (811; 911) for winding the suture.

18. The tissue closure device according to claim 17, wherein either:
the suture winding portion (811) is mounted in a recess structure (814) on the main body housing (810), the suture winding portion (811) comprises a mounting base portion (8111) directly joined to the main body housing (810) and a suture winding portion body (8112) located on the outer side of the mounting base portion (8111), the mounting base portion (8111) and the suture winding portion body (8112) are connected by a thin neck portion (8113), and the suture is wound around the thin neck portion (8113); or
a recessed groove extending circumferentially is provided on the sidewall of the main body housing (910) to form the suture winding portion (911).

19. The tissue closure device according to any one of claims 1-6, wherein the tissue closure device comprises a stabilizer (40) mounted at one end of the main body housing (10) and actuatable between a folded position and an unfolded position, and when the access needle is in the actuated position, the access needle (50) penetrates through the stabilizer (40) in the unfolded position.

## Patentansprüche

1. Gewebeverschlussvorrichtung (100), umfassend:
ein Grundkörpergehäuse (10; 610; 710; 810; 910; 1010) mit einer Längsachse, wobei das Grundkörpergehäuse (10; 610; 710; 810; 910; 1010) intern eine sich entlang der Längsachse erstreckende Kammer definiert, ein Nahtmaterialschlitz (12) an einer Seitenwand des Grundkörpergehäuses (10; 610; 710; 810; 910; 1010) bereitgestellt ist, und der Grundkörper ein Nadelaustrittsloch (16; 616; 716; 816; 916; 1016) definiert; und
eine Zugangsnadel (50; 1050), die in der Kammer vorgeladen werden kann, mit einem Nahtmaterialtrageabschnitt (51) nahe einer Spitze (52) davon bereitgestellt ist, in einer Anfangsposition gehalten werden kann und von der Anfangsposition zu einer betätigten Position distal betätigt werden kann,
wobei, wenn sich die Zugangsnadel (50; 1050) in der Anfangsposition befindet, die Zugangsnadel (50) in der Kammer aufgenommen ist und der Nahtmaterialtrageabschnitt (51) und der Nahtmaterialschlitz (12) mindestens teilweise miteinander in Verbindung stehen, um einem Nahtmaterial zu ermöglichen, über den Nahtmaterialschlitz (12) in den Nahtmaterialtrageabschnitt (51) einzutreten; und wenn sich die Zugangsnadel (50) in der betätigten Position befindet, die Zugangsnadel (50) das Nadelaustrittsloch (16; 616; 716; 816; 916; 1016) durchdringt, und die Spitze (52) der Zugangsnadel (50) sich außerhalb der Kammer befindet;
wobei der Nahtmaterialschlitz (12) einen Nahtmaterialbeladungsschlitz (121; 6121; 7121; 8121; 9121; 10121) umfasst, wobei der Nahtmaterialbeladungsschlitz (121; 6121; 7121; 8121; 9121; 10121) konfiguriert ist, um zu ermöglichen, dass das Nahtmaterial von außerhalb der Gewebeverschlussvorrichtung über den Nahtmaterialbeladungsschlitz (121; 6121; 7121; 8121; 9121; 10121) in den Nahtmaterialtrageabschnitt (51) der Zugangsnadel (50) geladen wird; und **dadurch gekennzeichnet, dass:**
die Nadelaustrittslöcher (16; 616; 716; 816; 916; 1016) an der Seitenwand des Grundkörpergehäuses (10; 610; 710; 810; 910; 1010) bereitgestellt sind; und
der Nahtmaterialschlitz (12) ferner einen Nahtmaterialfreigabeschlitz (122; 6122; 7122; 8122; 9122; 10122) umfasst, wobei der Nahtmaterialfreigabeschlitz (122; 6122; 7122; 8122; 9122; 10122) zwischen dem Nahtmaterialbeladungsschlitz (121; 6121; 7121; 8121; 9121; 10121) und dem Nadelaustrittsloch (16; 616; 716; 816; 916; 1016) in Verbindung steht und konfiguriert ist, um dem Nahtmaterial zu ermöglichen, sich von der Gewebeverschlussvorrichtung (100) über den Nahtmaterialfreigabeschlitz (122; 6122; 7122; 8122; 9122; 10122) zu lösen, wobei der Nahtmaterialbeladungsschlitz (121; 6121; 7121; 8121; 9121; 10121) und der Nahtmaterialtrageabschnitt (51) mindestens teilweise ausgerichtet sind, wenn sich die Zugangsnadel (50) in der Anfangsposition befindet.

2. Gewebeverschlussvorrichtung nach Anspruch 1, wobei der Nahtmaterialschlitz (1012) eine integrierte Schlitzstruktur ist, die sowohl als Nahtmaterialbeladungsschlitz (10121) als auch als Nahtmaterialfreigabeschlitz (10122) dient, wobei der Nahtmaterialschlitz (1012) konfiguriert ist, um zu ermöglichen, dass das Nahtmaterial von außerhalb der Gewebeverschlussvorrichtung in den Nahtmaterialtrageabschnitt der Zugangsnadel (1050) über den Nahtmaterialschlitz (1012) geladen wird und um zu ermöglichen, dass sich das Nahtmaterial von der Gewebeverschlussvorrichtung über den Nahtmaterialschlitz (1012) löst.

3. Gewebeverschlussvorrichtung nach Anspruch 1, wobei der Nahtmaterialbeladungsschlitz (121; 6121; 7121; 8121; 9121; 10121) einen ersten Schlitzabschnitt (1211; 61211) und einen zweiten Schlitzabschnitt (1212; 61212) aufweist, wobei sich der erste Schlitzabschnitt (1211; 61211) mindestens teilweise in Richtung der Achse von außen zu dem zweiten Schlitzabschnitt (1212; 61212) erstreckt, und sich der zweite Schlitzabschnitt (1212; 61212) mindestens teilweise axial von dem ersten Schlitzabschnitt (1211; 61211) entlang einer Richtung weg von dem Nadelaustrittsloch (16; 616; 716; 816; 916; 1016) erstreckt, wobei der zweite Schlitzabschnitt (1212; 61212) und der Nahtmaterialtrageabschnitt (51) mindestens teilweise aneinander ausgerichtet sind; wobei optional entweder:
der erste Schlitzabschnitt (1211; 61211) eine längliche Öffnung entlang einer Richtung bildet, die mindestens teilweise senkrecht zu der Längsachse verläuft, wobei zwei Spitzen der länglichen Öffnung sich jeweils entlang einer Richtung weg von dem Nadelaustrittsloch (16; 616; 716; 816; 916; 1016) und zu der Achse hin erstrecken, um gemeinsam den zweiten Schlitzabschnitt (1212; 61212) zu definieren; oder:
die Erstreckungsrichtungen des Nahtmaterialfreigabeschlitzes (122; 6122; 7122; 8122; 9122; 10122) und des zweiten Schlitzabschnitts (1212; 61212) gleich sind.

4. Gewebeverschlussvorrichtung nach Anspruch 1, wobei eine Schneidoberfläche (618), die eine Führungsstruktur bildet, an einem Einlass des Schlitzes (6121) an der Seitenwand des Grundkörpergehäuses (610) bereitgestellt ist.

5. Gewebeverschlussvorrichtung nach Anspruch 1, wobei das Nadelaustrittsloch (16; 616; 716; 816; 916; 1016) eine Öffnung ist, die der Form der Zugangsnadel (50) entspricht.

6. Gewebeverschlussvorrichtung nach Anspruch 1, wobei zwei perforierte Strukturen, die symmetrisch bezüglich der Längsachse sind, an der Seitenwand des Grundkörpergehäuses (10) bereitgestellt sind, wobei jede der perforierten Strukturen mindestens einen Nahtmaterialschlitz (12) und mindestens ein Nadelaustrittsloch (16; 616; 716; 816; 916; 1016) umfasst.

7. Gewebeverschlussvorrichtung nach einem der Ansprüche 1 bis 6, wobei das Grundkörpergehäuse (610; 710) einen nicht-starren Nahtmaterialrückhalteabschnitt (660; 760) umfasst, der konfiguriert ist, um das Nahtmaterial kontaktieren zu können und das Nahtmaterial durch Reibung an Ort und Stelle zu halten.

8. Gewebeverschlussvorrichtung nach Anspruch 7, wobei das Grundkörpergehäuse (610) eine starre Seitenwand mit einem darauf ausgebildeten Fenster umfasst, wobei der Nahtmaterialrückhalteabschnitt (660) an dem Fenster angebracht ist und einen Teil der Seitenwand des Grundkörpergehäuses (610) bildet, wobei mindestens ein Teil des Nahtmaterialschlitzes mindestens an dem Nahtmaterialrückhalteabschnitt (660) ausgebildet ist.

9. Gewebeverschlussvorrichtung nach Anspruch 8, wobei der Nahtmaterialschlitz einen Nahtmaterialbeladungsschlitz (6121) und einen Nahtmaterialfreigabeschlitz (6122) umfasst, der zwischen dem Nahtmaterialbeladungsschlitz (6121) und dem Nadelaustrittsloch (616) in Verbindung steht, wobei:
mindestens ein Teil des Nahtmaterialbeladungsschlitzes (6121) an dem Nahtmaterialrückhalteabschnitt (660) ausgebildet ist; und/oder
mindestens ein Teil des Nahtmaterialfreigabeschlitzes (6122) an dem Nahtmaterialrückhalteabschnitt (660) ausgebildet ist.

10. Gewebeverschlussvorrichtung nach Anspruch 8, wobei entweder:
ein Vorsprungsabschnitt (661), der entlang der Seitenwand des Grundkörpergehäuses (610) vorsteht, an dem Nahtmaterialrückhalteabschnitt (660) angeordnet ist, ein entsprechender Vertiefungsabschnitt an dem Fenster der starren Seitenwand bereitgestellt ist, und der Vorsprungsabschnitt (661) und der Vertiefungsabschnitt zusammenwirken, um den Nahtmaterialrückhalteabschnitt (660) und die starre Seitenwand zu sichern; oder
ein Vorsprungsabschnitt, der entlang der Seitenwand des Grundkörpergehäuses vorsteht, an dem Fenster der starren Seitenwand bereitgestellt ist, ein entsprechender Vertiefungsabschnitt an dem Rand des Nahtmaterialrückhalteabschnitts bereitgestellt ist, und der Vorsprungsabschnitt und der Vertiefungsabschnitt zusammenwirken, um den Nahtmaterialrückhalteabschnitt und die starre Seitenwand zu sichern.

11. Gewebeverschlussvorrichtung nach Anspruch 8, wobei ein als Nahtmaterialbeladungsschlitz (6121) dienender Abschnitt des Nahtmaterialschlitzes (612) mindestens teilweise an dem Nahtmaterialrückhalteabschnitt (660) ausgebildet ist und eine Breite aufweist, die kleiner oder gleich der Breite des Nahtmaterials ist.

12. Gewebeverschlussvorrichtung nach Anspruch 7, wobei das Grundkörpergehäuse (710) eine starre Seitenwand umfasst, der Nahtmaterialrückhalteabschnitt (760) an der starren Seitenwand angebracht ist und unabhängig von dem Nahtmaterialschlitz (712) ist, und der Nahtmaterialrückhalteabschnitt (760) konfiguriert ist, um dem Nahtmaterial zu ermöglichen, zwischen den Nahtmaterialrückhalteabschnitt (760) und die starre Seitenwand einzutreten, um das Nahtmaterial zwischen dem Nahtmaterialrückhalteabschnitt (760) und der starren Seitenwand zu klemmen.

13. Gewebeverschlussvorrichtung nach Anspruch 12, wobei der Nahtmaterialrückhalteabschnitt (760) an der Seite des Nahtmaterialbeladungsschlitzes (7121) angebracht ist, die von dem Nadelaustrittsloch (716) abgewandt ist; und optional wobei
zwei Nahtmaterialschlitzstrukturen an der Seitenwand des Grundkörpergehäuses (710) bereitgestellt sind, jede der Nahtmaterialschlitzstrukturen mindestens einen Nahtmaterialschlitz umfasst, und der Nahtmaterialrückhalteabschnitt (760) zwischen den zwei Nahtmaterialschlitzstrukturen angeordnet ist.

14. Gewebeverschlussvorrichtung nach Anspruch 12, wobei ein vertiefter Abschnitt (711) an der starren Seitenwand bereitgestellt ist, der Nahtmaterialrückhalteabschnitt (760) in den vertieften Abschnitt (711) eingesetzt ist, wobei dessen Form an denselben angepasst ist, und die Außenoberfläche des Nahtmaterialrückhalteabschnitts (760) bündig mit der Außenoberfläche der starren Seitenwand ist; und optional wobei:
der Nahtmaterialrückhalteabschnitt (760) einen zentralen Abschnitt (761) und Endabschnitte (762) umfasst, die an beiden Enden des zentralen Abschnitts (761) angeordnet sind, wobei die Innenoberfläche des zentralen Abschnitts (761) weiter nach innen in Richtung der Längsachse vorsteht als die Innenoberflächen der Endabschnitte (762).

15. Gewebeverschlussvorrichtung nach einem der Ansprüche 1 bis 6, wobei der Nahtmaterialschlitz (12) an der starren Seitenwand des Grundkörpergehäuses (10) ausgebildet ist, und der als Nahtmaterialbeladungsschlitz (121; 7121; 8121; 9121; 10121) dienende Abschnitt des Nahtmaterialschlitzes (12) eine Breite aufweist, die größer oder gleich der Breite des Nahtmaterialtrageabschnitts (51) der Zugangsnadel (50) ist.

16. Gewebeverschlussvorrichtung nach Anspruch 17, wobei die Begrenzung des Nahtmaterialbeladungsschlitzes (121) ein aufgeweitetes Profil (1212a) relativ zur Mitte des Nahtmaterialbeladungsschlitzes (121) aufweist; und optional wobei
das aufgeweitete Profil (1212a) ein gekrümmtes Profil ist.

17. Gewebeverschlussvorrichtung nach einem der Ansprüche 1 bis 6, wobei ein vertiefter und nach außen offener Nahtmaterialaufnahmeabschnitt (813; 913), der sich parallel zu der Längsachse erstreckt, an der Seitenwand des Grundkörpergehäuses (810; 910) bereitgestellt ist, wobei der Nahtmaterialaufnahmeabschnitt (813; 913) zwischen dem Nahtmaterialschlitz (8121; 9121) und einem Nahtmaterialwickelabschnitt (811; 911) zum Wickeln des Nahtmaterials verbunden ist.

18. Gewebeverschlussvorrichtung nach Anspruch 17, wobei entweder:
der Nahtmaterialwickelabschnitt (811) in einer Vertiefungsstruktur (814) an dem Grundkörpergehäuse (810) angebracht ist, der Nahtmaterialwickelabschnitt (811) einen Montagebasisteil (8111) umfasst, der direkt mit dem Grundkörpergehäuse (810) verbunden ist, und einen Nahtmaterialwickelabschnittskörper (8112), der sich auf der Außenseite des Montagebasisteils (8111) befindet, der Montagebasisteil (8111) und der Nahtmaterialwickelabschnittskörper (8112) durch einen dünnen Halsabschnitt (8113) verbunden sind, und die Naht um den dünnen Halsabschnitt (8113) gewickelt ist; oder
eine umlaufend verlaufende vertiefte Nut an der Seitenwand des Grundkörpergehäuses (910) bereitgestellt ist, um den Nahtmaterialwicklungsabschnitt (911) zu bilden.

19. Gewebeverschlussvorrichtung nach einem der Ansprüche 1 bis 6, wobei die Gewebeverschlussvorrichtung einen Stabilisator (40) umfasst, der an einem Ende des Grundkörpergehäuses (10) angebracht ist und zwischen einer gefalteten Position und einer entfalteten Position betätigbar ist, und wenn sich die Zugangsnadel in der betätigten Position befindet, die Zugangsnadel (50) den Stabilisator (40) in der entfalteten Position durchdringt.

## Revendications

1. Dispositif de fermeture de tissu (100) comprenant :
un logement de corps principal (10 ; 610 ; 710 ; 810 ; 910 ; 1010) ayant un axe longitudinal, le logement de corps principal (10 ; 610 ; 710 ; 810 ; 910 ; 1010) définissant de façon interne une chambre s'étendant le long de l'axe longitudinal, une fente de suture (12) étant fournie sur une paroi latérale du logement de corps principal (10 ; 610 ; 710 ; 810 ; 910 ; 1010), et le corps principal définissant un trou de sortie d'aiguille (16 ; 616 ; 716 ; 816 ; 916 ; 1016) ; et
une aiguille d'accès (50 ; 1050), qui est capable d'être préchargée dans la chambre, est pourvue d'une partie de support de suture (51) près d'une pointe (52) de celle-ci, est capable d'être maintenue dans une position initiale, et est capable d'être actionnée de manière distale de la position initiale à une position actionnée,
dans lequel lorsque l'aiguille d'accès (50 ; 1050) est dans la position initiale, l'aiguille d'accès (50) est reçue dans la chambre, et la partie de support de suture (51) et la fente de suture (12) sont au moins partiellement en communication pour permettre à une suture d'entrer dans la partie de support de suture (51) par l'intermédiaire de la fente de suture (12) ; et lorsque l'aiguille d'accès (50) est dans la position actionnée, l'aiguille d'accès (50) pénètre à travers le trou de sortie d'aiguille (16 ; 616 ; 716 ; 816 ; 916 ; 1016), et la pointe (52) de l'aiguille d'accès (50) est localisée à l'extérieur de la chambre ;
dans lequel la fente de suture (12) comprend une fente de chargement de suture (121 ; 6121 ; 7121 ; 8121 ; 9121 ; 10121), la fente de chargement de suture (121 ; 6121 ; 7121 ; 8121 ; 9121 ; 10121) étant conçue pour permettre à la suture d'être chargée depuis l'extérieur du dispositif de fermeture de tissu dans la partie de support de suture (51) de l'aiguille d'accès (50) par l'intermédiaire de la fente de chargement de suture (121 ; 6121 ; 7121 ; 8121 ; 9121 ; 10121) ; et **caractérisé en ce que :**
le trou de sortie d'aiguille (16 ; 616 ; 716 ; 816 ; 916 ; 1016) est fourni sur la paroi latérale du logement de corps principal (10 ; 610 ; 710 ; 810 ; 910 ; 1010) ; et
la fente de suture (12) comprend en outre une fente de libération de suture (122 ; 6122 ; 7122 ; 8122 ; 9122 ; 10122), la fente de libération de suture (122 ; 6122 ; 7122 ; 8122 ; 9122 ; 10122) étant en communication entre la fente de chargement de suture (121 ; 6121 ; 7121 ; 8121 ; 9121 ; 10121) et le trou de sortie d'aiguille (16 ; 616 ; 716 ; 816 ; 916 ; 1016) et étant conçue pour permettre à la suture de se désaccoupler du dispositif de fermeture de tissu (100) par l'intermédiaire de la fente de libération de suture (122 ; 6122 ; 7122 ; 8122 ; 9122 ; 10122), dans lequel la fente de chargement de suture (121 ; 6121 ; 7121 ; 8121 ; 9121 ; 10121) et la partie de support de suture (51) sont au moins partiellement alignées lorsque l'aiguille d'accès (50) est dans la position initiale.

2. Dispositif de fermeture de tissu selon la revendication 1, dans lequel la fente de suture (1012) est une structure de fente intégrée servant à la fois de fente de chargement de suture (10121) et de fente de libération de suture (10122), la fente de suture (1012) étant conçue pour permettre à la suture d'être chargée depuis l'extérieur du dispositif de fermeture de tissu dans la partie de support de suture de l'aiguille d'accès (1050) par l'intermédiaire de la fente de suture (1012) et pour permettre à la suture de se désaccoupler du dispositif de fermeture de tissu par l'intermédiaire de la fente de suture (1012).

3. Dispositif de fermeture de tissu selon la revendication 1, dans lequel la fente de chargement de suture (121 ; 6121 ; 7121 ; 8121 ; 9121 ; 10121) a une première partie de fente (1211 ; 61211) et une seconde partie de fente (1212 ; 61212), la première partie de fente (1211 ; 61211) s'étendant au moins partiellement en direction de l'axe allant de l'extérieur à la seconde partie de fente (1212 ; 61212), et la seconde partie de fente (1212 ; 61212) s'étendant au moins partiellement axialement à partir de la première partie de fente (1211 ; 61211) le long d'une direction à l'écart du trou de sortie d'aiguille (16 ; 616 ; 716 ; 816 ; 916 ; 1016), dans lequel la seconde partie de fente (1212 ; 61212) et la partie de support de suture (51) sont au moins partiellement alignées ; facultativement dans lequel soit :
la première partie de fente (1211 ; 61211) constitue une ouverture allongée le long d'une direction au moins partiellement perpendiculaire à l'axe longitudinal, deux pointes de l'ouverture allongée s'étendant respectivement le long d'une direction à l'écart du trou de sortie d'aiguille (16 ; 616 ; 716 ; 816 ; 916 ; 1016) et en direction de l'axe pour définir conjointement la seconde partie de fente (1212 ; 61212) ; soit :
les directions d'extension de la fente de libération de suture (122 ; 6122 ; 7122 ; 8122 ; 9122 ; 10122) et de la seconde partie de fente (1212 ; 61212) sont identiques.

4. Dispositif de fermeture de tissu selon la revendication 1, dans lequel une surface de découpe (618) qui constitue une structure de guidage est fournie au niveau d'une entrée de la fente (6121) sur la paroi latérale du logement de corps principal (610).

5. Dispositif de fermeture de tissu selon la revendication 1, dans lequel le trou de sortie d'aiguille (16 ; 616 ; 716 ; 816 ; 916 ; 1016) est une ouverture s'adaptant à la forme de l'aiguille d'accès (50).

6. Dispositif de fermeture de tissu selon la revendication 1, dans lequel deux structures perforées qui sont symétriques autour de l'axe longitudinal sont fournies sur la paroi latérale du logement de corps principal (10), chacune des structures perforées comprenant au moins une fente de suture (12) précitée et au moins un trou de sortie d'aiguille (16 ; 616 ; 716 ; 816 ; 916 ; 1016) précité.

7. Dispositif de fermeture de tissu selon l'une quelconque des revendications 1 à 6, dans lequel le logement de corps principal (610 ; 710) comprend une partie de retenue de suture (660 ; 760) non rigide conçue pour être capable de venir en contact avec la suture et retenant la suture en place par friction.

8. Dispositif de fermeture de tissu selon la revendication 7, dans lequel le logement de corps principal (610) comprend une paroi latérale rigide ayant une fenêtre formée sur celle-ci, la partie de retenue de suture (660) étant montée au niveau de la fenêtre et constituant une partie de la paroi latérale du logement de corps principal (610), dans lequel au moins une partie de la fente de suture est au moins formée sur la partie de retenue de suture (660).

9. Dispositif de fermeture de tissu selon la revendication 8, dans lequel la fente de suture comprend une fente de chargement de suture (6121), et une fente de libération de suture (6122) en communication entre la fente de chargement de suture (6121) et le trou de sortie d'aiguille (616), dans lequel :
au moins une partie de la fente de chargement de suture (6121) est formée sur la partie de retenue de suture (660) ; et/ou
au moins une partie de la fente de libération de suture (6122) est formée sur la partie de retenue de suture (660).

10. Dispositif de fermeture de tissu selon la revendication 8, dans lequel soit :
une partie de saillie (661) faisant saillie le long de la paroi latérale du logement de corps principal (610) est disposée sur la partie de retenue de suture (660), une partie d'évidement correspondante est fournie au niveau de la fenêtre de la paroi latérale rigide, et la partie de saillie (661) et la partie d'évidement coopèrent pour fixer la partie de retenue de suture (660) et la paroi latérale rigide ; soit
une partie de saillie faisant saillie le long de la paroi latérale du logement de corps principal est fournie au niveau de la fenêtre de la paroi latérale rigide, une partie d'évidement correspondante est fournie au niveau du bord de la partie de retenue de suture, et la partie de saillie et la partie d'évidement coopèrent pour fixer la partie de retenue de suture et la paroi latérale rigide.

11. Dispositif de fermeture de tissu selon la revendication 8, dans lequel une partie servant de fente de chargement de suture (6121) de la fente de suture (612) est au moins partiellement formée sur la partie de retenue de suture (660), et a une largeur inférieure ou égale à la largeur de la suture.

12. Dispositif de fermeture de tissu selon la revendication 7, dans lequel le logement de corps principal (710) comprend une paroi latérale rigide, la partie de retenue de suture (760) est montée sur la paroi latérale rigide et est indépendante de la fente de suture (712), et la partie de retenue de suture (760) est conçue pour permettre à la suture d'entrer entre la partie de retenue de suture (760) et la paroi latérale rigide de façon à serrer la suture entre la partie de retenue de suture (760) et la paroi latérale rigide.

13. Dispositif de fermeture de tissu selon la revendication 12, dans lequel la partie de retenue de suture (760) est montée sur le côté de la fente de chargement de suture (7121) à l'écart du trou de sortie d'aiguille (716) ; et facultativement dans lequel
deux structures de fente de suture sont fournies sur la paroi latérale du logement de corps principal (710), chacune des structures de fente de suture comprend au moins une fente de suture précitée, et la partie de retenue de suture (760) est localisée entre les deux structures de fente de suture.

14. Dispositif de fermeture de tissu selon la revendication 12, dans lequel une partie évidée (711) est fournie sur la paroi latérale rigide, la partie de retenue de suture (760) est insérée dans la partie évidée (711) avec la forme de celle-ci qui lui est adaptée, et la surface externe de la partie de retenue de suture (760) est affleurante à la surface externe de la paroi latérale rigide ; et facultativement dans lequel :
la partie de retenue de suture (760) comprend une partie centrale (761) et des parties d'extrémité (762) localisées au niveau de l'une et l'autre des extrémités de la partie centrale (761), la surface interne de la partie centrale (761) faisant saillie plus loin vers l'intérieur en direction de l'axe longitudinal par rapport aux surfaces internes des parties d'extrémité (762).

15. Dispositif de fermeture de tissu selon l'une quelconque des revendications 1 à 6, dans lequel la fente de suture (12) est formée sur la paroi latérale rigide du logement de corps principal (10), et la partie servant de fente de chargement de suture (121 ; 7121 ; 8121 ; 9121 ; 10121) de la fente de suture (12) a une largeur supérieure ou égale à la largeur de la partie de support de suture (51) de l'aiguille d'accès (50).

16. Dispositif de fermeture de tissu selon la revendication 17, dans lequel la limite de la fente de chargement de suture (121) a un profil évasé (1212a) par rapport au centre de la fente de chargement de suture (121) ; et facultativement dans lequel
le profil évasé (1212a) est un profil courbé.

17. Dispositif de fermeture de tissu selon l'une quelconque des revendications 1 à 6, dans lequel une partie de réception de suture (813 ; 913) évidée et ouverte vers l'extérieur qui s'étend parallèle à l'axe longitudinal est fournie sur la paroi latérale du logement de corps principal (810 ; 910), la partie de réception de suture (813 ; 913) étant reliée entre la fente de suture (8121 ; 9121) et une partie d'enroulement de suture (811 ; 911) permettant d'enrouler la suture.

18. Dispositif de fermeture de tissu selon la revendication 17, dans lequel soit :
la partie d'enroulement de suture (811) est montée dans une structure d'évidement (814) sur le logement de corps principal (810), la partie d'enroulement de suture (811) comprend une partie de base de montage (8111) jointe directement au logement de corps principal (810) et un corps de partie d'enroulement de suture (8112) localisé sur le côté externe de la partie de base de montage (8111), la partie de base de montage (8111) et le corps de partie d'enroulement de suture (8112) sont reliés par une mince partie de col (8113), et la suture est enroulée autour de la mince partie de col (8113) ; soit
une rainure évidée s'étendant circonférentiellement est fournie sur la paroi latérale du logement de corps principal (910) pour former la partie d'enroulement de suture (911).

19. Dispositif de fermeture de tissu selon l'une quelconque des revendications 1 à 6, dans lequel le dispositif de fermeture de tissu comprend un stabilisateur (40) monté au niveau d'une extrémité du logement de corps principal (10) et actionnable entre une position pliée et une position dépliée, et lorsque l'aiguille d'accès est dans la position actionnée, l'aiguille d'accès (50) pénètre à travers le stabilisateur (40) dans la position dépliée.
